# EUROPEAN PATENT SPECIFICATION

(11) **EP 1 278 860 B1**
(45) Date of publication and mention of the grant of the patent: **11.05.2005**
(21) Application number: 01929487.5
(22) Date of filing: 31.03.2001
(51) Int. Cl.: C12N 15/54, C12N 9/10, C12N 1/21, C12P 13/08, C12P 13/14, C12Q 1/68

(54) **NUCLEOTIDE SEQUENCES WHICH CODE FOR THE CMA GENE**
FÜR DAS CMA-GEN CODIERENDE NUKLEOTIDSEQUENZEN
SEQUENCES NUCLEOTIDIQUES CODANT POUR LE GENE DE LA CMA

(30) Priority: 04.05.2000 DE 10021832
(43) Date of publication of application: 29.01.2003
(73) Proprietor: Degussa AG, 40474 Düsseldorf (DE); Forschungszentrum Jülich GmbH, 52425 Jülich (DE)
(72) Inventor: NAMPOOTHIRI, Madhavan, Quilon (Dist), PIN691 Kerala (IN); MÖCKEL, Bettina, 40597 Düsseldorf (DE); EGGELING, Lothar, 52428 Jülich (DE); SAHM, Hermann, 52428 Jülich (DE)
(86) International application number: PCT/EP2001/003703
(87) International publication number: WO 2001/083764

(56) References cited:
- EP-A- 0 771 879
- EP-A- 1 108 790
- WO-A-01/00805
- US-A- 3 784 446
- US-A- 5 770 409
- EGGELING L. AND SAHM H.: "L-Glutamate and L-Lysine: traditional products with impetuous developments" APPLIED MICROBIOLOGY AND BIOTECHNOLOGY, vol. 52, August 1999 (1999-08), pages 146-153, XP000979507 ISSN: 0175-7598
- EIKMANNS B.J. ET AL: 'Molecular aspects of lysine, threonine, and isoleucine biosynthesis in Corynebacterium glutamicum' ANTONIE VAN LEEUWENHOEK vol. 64, 1993, pages 145 - 163
- INSTITUT NATIONAL POLYTECHNIQUE DE LORRAINE - Laboratoire des Sciences du Génie Chimique. These présentée par MYINT - SEIN en vue d'obtenir le titre de DOCTEUR de L'INPL - Spécialité: Biotechnologies et Industries Alimentaires. Sujet: Etudes cinétiques et modélisation de la fermentation de lysine pa
- NAMPOOTHIRI K.M. ET AL: 'Expression of genes of lipid synthesis and altered lipid composition modulates L-glutamate efflux of Corynebacterium glutamicum' APPLIED MICROBIOLOGY AND BIOTECHNOLOGY vol. 58, no. 1, 2002, pages 89 - 96

## Description

The invention provides genetically modified coryneform bacteria, comprising amplified nucleotide sequences which code for cyclopropane-mycolic acid synthase and a process for the fermentative preparation of L-lysine and L-glutamate using coryneform bacteria in which the cma gene, which codes for cyclopropane-mycolic acid synthase, is amplified.

Amino acids, in particular L-lysine and L-glutamate, are used in human medicine, in the pharmaceuticals industry, in the foodstuffs industry, but in particular. in animal nutrition.

It is known that amino acids are prepared by fermentation from strains of coryneform bacteria, in particular *Corynebacterium glutamicum.* Because of their great importance, work is constantly being undertaken to improve the preparation processes. Improvements to the processes can relate to fermentation measures, such as e. g. stirring and supply of oxygen, or the composition of the nutrient media, such as e. g. the sugar concentration during the fermentation, or the working up to the product form by e. g. ion exchange chromatography, or the intrinsic output properties of the microorganism itself.

Methods of mutagenesis, selection and mutant selection are used to improve the output properties of these microorganisms. Strains which are resistant to antimetabolites, such as e. g. the lysine analogue S-(2-aminoethyl)-cysteine, or are auxotrophic for metabolites of regulatory importance and produce L-amino acids, such as e. g. L-lysine or L-glutamate, are obtained in this manner.

Methods of the recombinant DNA technique have also been employed for some years for improving the strain of *Corynebacterium* strains which produce amino acids, by amplifying individual amino acid biosynthesis genes and investigating the effect on the amino acid production. Review articles in this context are to be found, inter alia, in Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten and Sinskey (Critical Reviews in Biotechnology 15, 73-103 (1995)) and Sahm et al. (Annuals of the New York Academy of Science 782, 25-39 (1996))..

EP-A-0771879 shows a genetically modified coryneform bacterium deficient in α-ketoglutarate dehydrogenase acitivity, the nucleotide sequence of the gene encoding said α-ketoglutarate dehydrogenase and the corresponding amino acid sequence, and a process for the fermentative preparation of L-glutamic acid by means of said bacterium. Eggeling L. and Sahm H. describe in Appl. Microbiol. Biotechnol. (1992) 52: 146-153 the principles of how carbon metabolism is engineered in central reations and biosynthesis pathways in L-glutamate and L-lysine producing Corynebacterium glutamicum.

The object of the present invention was to provide new aids for improved fermentative preparation of L-lysine and L-glutamate.

This object is achieved by providing a genetically modified coryneform bacterium, the cma gene of which, which codes for cyclopropane-mycolic acid synthase, is amplified.

Amino acids, in particular L-lysine and L-glutamate, are used in human medicine, in the pharmaceuticals industry, in the foodstuffs industry, and in particular in animal nutrition. There is therefore a general interest in providing new improved processes for the preparation of L-lysine and L-glutamate.

When L-lysine or lysine and L-glutamate or glutamate are mentioned in the following, not only the bases but also the salts, such as e. g. lysine monohydrochloride or lysine sulfate, are also meant by this.

The invention provides a genetically modified coryneform bacterium, in which its cma gene, which codes for cyclopropane-mycolic acid synthase, is amplified.

The term "amplification" in this connection describes the increase in intracellular activity of one or more enzymes in a microorganism which are coded by the corresponding DNA.

Amplification can be achieved with the aid of various manipulations of the bacterial cell.

To achieve an amplification, in particular an over-expression, the number of copies of the corresponding genes can be increased, a potent promoter can be used, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-lysine or L-glutamate production. It is also possible to use a gene which codes for a corresponding enzyme with a high activity. The expression is likewise improved by measures to prolong the life of the m-RNA. Furthermore, the enzyme activity is also increased overall by preventing the degradation of the enzyme. These measures can optionally also be combined as desired.

The microorganisms which the present invention provides can prepare L-amino acids, in particular L-lysine and L-glutamate, from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species *Corynebacterium glutamicum*, which is known among experts for its ability to produce L-amino acids.

Suitable strains of the genus Corynebacterium, in particular of the species *Corynebacterium glutamicum,* are, for example, the known wild-type strains
Corynebacterium glutamicum ATCC13032 .
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 and
Brevibacterium divaricatum ATCC14020
and L-lysine-producing mutants or strains prepared therefrom, such as, for example
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 and
Corynebacterium glutamicum DSM5715.

The present description provides an isolated polynucleotide from coryneform bacteria, comprising a polynucleotide sequence chosen from the group consisting of
a) polynucleotide which is identical to a polynucleotide which codes for a polypeptide which comprises the amino acid sequence of SEQ ID No. 2,
b) polynucleotide which codes for a polypeptide which comprises an amino acid sequence which is identical to the extent of at least 90% with the amino acid sequence of SEQ ID. No. 2,
c) polynucleotide which is complementary to the polynucleotides of a) or b), and
d) polynucleotide comprising at least 15 successive nucleotides of the polynucleotide sequence of a), b) or c).

In the context of the present Application, a polynucleotide sequence is "homologous" to the sequence according to the invention if it coincides in its base composition and sequence with the sequence according to the invention to the extent of at least 90%. According to the present invention, a "homologous protein" is to be understood as proteins which have an amino acid sequence which coincides with the amino acid sequence coded by the cma gene (SEQ ID No.1) to the extent of at least 90 %, "coincide" being understood as meaning that either the corresponding amino acids are identical or they are amino acids which are homologous to one another. Those amino acids which correspond in their properties, in particular in respect of charge, hydrophobicity, steric properties etc., are called "homologous amino acids".

The description also provides a polynucleotide as described above , this preferably being a DNA which is capable of replication, comprising:
(i) the nucleotide sequence shown in SEQ ID no. 1, or
(ii) at least one sequence which corresponds to sequence (i) in the context of the degeneration of the genetic code, or
(iii) at least one sequence which hybridizes with the sequence complementary to sequence (i) or (ii).

The description also provides
a polynucleotide which is capable of replication and comprises the nucleotide sequence SEQ ID No. 1, or consists of it,
a polynucleotide sequence which codes for a polypeptide which comprises the amino acid sequence SEQ ID No. 2, or consists of it.

The invention provides:
a) a genetically modified coryneform bacterium in which a polynucleotide encoding a polypeptide having an amino acid sequence which is at least 90% identical to the amino acid sequence set out in SEQ ID NO: 2 is amplified, wherein the polypeptide has the activity of cyclopropane-mycolic acid synthase, and which produce L-lysin or L-glutamate in an improved manner in relation to the corresponding non-genetically modified coryneform bacterium.
b) The bacterium of claim 1, wherein said polynucleotide is overexpressed:
c) The bacterium of claim 1, wherein said polypeptide has the amino acid sequence set out in SEQ ID NO: 2.
d) Coryneform bacterium according to claims 1 or 2, especially of genus Corynebacterium glutamicum, wherein the polynucleotide is chosen from the group:
   a) nucleotide sequence as set out in SEQ ID NO: 1; or
   b) nucleotide sequences corresponding to sequence SEQ ID NO: 1 within the degeneracy of the genetic code.

The description provides, too:
a vector containing the DNA sequence of *C. glutamicum* which codes for the cma gene, contained in the vector (plasmid) pJC1cma, deposited in *Corynebacterium glutamicum* under number DSM 13248,
and coryneform bacteria serving as the host cell, which contain the vector.

The description also provides polynucleotides which comprise the complete gene with the polynucleotide sequence corresponding to SEQ ID No. 1 or fragments thereof, and which are obtainable by screening by means of hybridization of a corresponding gene library with a probe which comprises the sequence of the polynucleotide mentioned, according to SEQ ID No. 1, or a fragment thereof, and isolation of the DNA sequence mentioned.

Polynucleotide sequences according to the description are also suitable as hybridization probes for RNA, cDNA and DNA, in order to isolate, in the full length, cDNA which codes for cyclopropane-mycolic acid synthase and to isolate. those cDNA or genes which have a high similarity with the sequence of the cyclopropane-mycolic acid synthase gene.

Polynucleotide sequences according to the description are furthermore suitable as primers for the polymerase chain reaction (PCR), for the preparation of DNA which codes for cyclopropane-mycolic acid synthases.

Such oligonucleotides which serve as probes or primers can comprise more than 30, preferably up to 30, particularly preferably up to 20, especially preferably at least 15 successive nucleotides. Oligonucleotides which have a length of at least 40. or 50 nucleotides are also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

"Polypeptides" is understood as meaning peptides or proteins which comprise two or more amino acids bonded via peptide bonds.

The polypeptides according to the description include a polypeptide according to SEQ ID No. 2, with the biological activity of cyclopropane-mycolic acid synthase and also those which are identical to the extent of at least 90 to 95% with the polypeptide according to SEQ ID no. 2, and have the activity mentioned.

The invention moreover provides a process for the fermentative preparation of L-lysine and L-glutamate using coryneform bacteria which in particular already produce said amino acids, and in which the nucleotide sequences which code for the cma gene are amplified, in particular over-expressed.

The cma gene of *C. glutamicum* codes for cyclopropane-mycolic acid synthase.

To isolate the cma gene or also other genes of *C. glutamicum*, a gene library of this microorganism was first set up in *E. coli*. The setting up of gene libraries is described in generally known textbooks and handbooks. The textbook by Winnacker: Gene und Klone, Eine Einführung in die Gentechnologie [Genes and Clones, An Introduction to Genetic Engineering] (Verlag Chemie, Weinheim, Germany, 1990) or the handbook by Sambrook,et al.: Molecular Cloning, A Laboratory Manual (Cold Spring Harbor Laboratory Press, 1989) may be mentioned as an example. A well-known gene library is that of the *E. coli* K-12 strain W3110 set up in λ vectors by Kohara et al. (Cell 50, 495-508 (1987)). Bathe et al. (Molecular and General Genetics, 252:255-265, 1996) describe a gene library of *C. glutamicum* ATCC13032, which was set up with the aid of the cosmid vector SuperCos I (Wahl et al., 1987, Proceedings of the National Academy of Sciences USA, 84:2160-2164) in the *E. coli* K-12 strain NM554 (Raleigh et al., 1988, Nucleic Acids Research 16:1563-1575). Börmann et al. (Molecular Microbiology 6(3), 317-326)) (1992)) in turn describe a gene library of *C. glutamicum* ATCC13032 using the cosmid pHC79 (Hohn and Collins, Gene 11, 291-298 (1980)). To prepare a gene library of *C. glutamicum* in *E. coli* it is also possible to use plasmids such as pBR322 (Bolivar, Life Sciences, 25, 807-818 (1979)) or pUC9 (Vieira et al., 1982, Gene, 19:259-268). Suitable hosts are, in particular, those *E. coli* strains which are restriction- and recombination-defective. An example of these is the strain DH5αmcr, which has been described by Grant et. al. (Proceedings of the National Academy of Sciences USA, 87 (1990) 4645-4649) The long DNA fragments cloned with the aid of cosmids can then in turn be subcloned and subsequently sequenced in the usual vectors which are suitable for sequencing, such as is described e. g. by Sanger et al. (Proceedings of the National Academy of Sciences of the United States of America, 74:5463-5467, 1977).

The DNA sequence of *C. glutamicum* which codes for the cma gene and which, as SEQ ID No. 1 was obtained in this manner. The amino acid sequence of the corresponding protein has moreover been derived from the present DNA sequence by the methods described above. The resulting amino acid sequence of the cma gene product is shown in SEQ ID No. 2.

Coding DNA sequences which result from SEQ ID No. 1 by the degeneracy of the genetic code are also a constituent of the description. In the same way, DNA sequences which hybridize with SEQ ID No. 1 or parts of SEQ ID No. 1 are a constituent of the description. Conservative amino acid exchanges, such as e. g. exchange of glycine for alanine or of aspartic acid for glutamic acid in proteins, are moreover known among experts as "sense mutations" which do not lead to a fundamental change in the activity of the protein, i.e. are of neutral function. It is moreover known that changes on the N and/or C terminus of a protein cannot substantially impair the function thereof or can even stabilize this. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the description.

In the same way, DNA sequences which hybridize with SEQ ID No. 1 or parts of SEQ ID No. 1 are a constituent of the description. Finally, DNA sequences which are prepared by the polymerase chain reaction (PCR) using olognucleotide [sic] primers which result from SEQ ID NO. 1 are a constituent of the description. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology (1991) 41: 255-260). Instructions for amplification of DNA sequences with the aid of the polymerase chain reaction (PCR) can be found by the expert, inter alia, in the handbook by Gait: Oligonukleotide [sic] synthesis: a practical approach (IRL Press, Oxford, UK, 1984) and in Newton and Graham: PCR (Spektrum Akademischer Verlag, Heidelberg, Germany, 1994).

In the work on the present invention, it has been found that coryneform bacteria produce L-lysine and L-glutamate in an improved manner after amplification of the cma gene.

The genes or gene constructs under consideration can either be present in plasmids with a varying number of copies, or can be integrated and amplified in the chromosome. Alternatively, an over-expression of the genes in question can moreover be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification EPS 0 472 869, in US Patent 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15 - 24 (1993)), in Japanese Laid-Open Specification JP-A-10-229891, in Jensen and Hammer (Biotechnology and Bioengineering 58, 191-195 (1998)), in Makrides (Microbiological Reviews 60:512-538 (1996)) and in known textbooks of genetics and molecular biology.

By way of example, the cma gene was over-expressed with the aid of plasmids.

Suitable plasmids are those which are replicated and expressed in coryneform bacteria. Numerous known plasmid vectors, such as e. g. pZ1 (Menkel et al., Applied and Environmental Microbiology (1989) 64: 549-554), pEKEx1 (Eikmanns et al., Gene 102:93-98 (1991)) or pHS2-1 (Sonnen et al., Gene 107:69-74 (1991)) are based on the cryptic plasmids pHM1519, pBLl or pGA1. Other plasmid vectors, such as e. g. those based on pCG4 (US-A 4,489,160), or pNG2 (Serwold-Davis et al., FEMS Microbiology Letters 66, 119-124 (1990)), or pAG1 (US-A 5,158,891), can be used in the same manner.

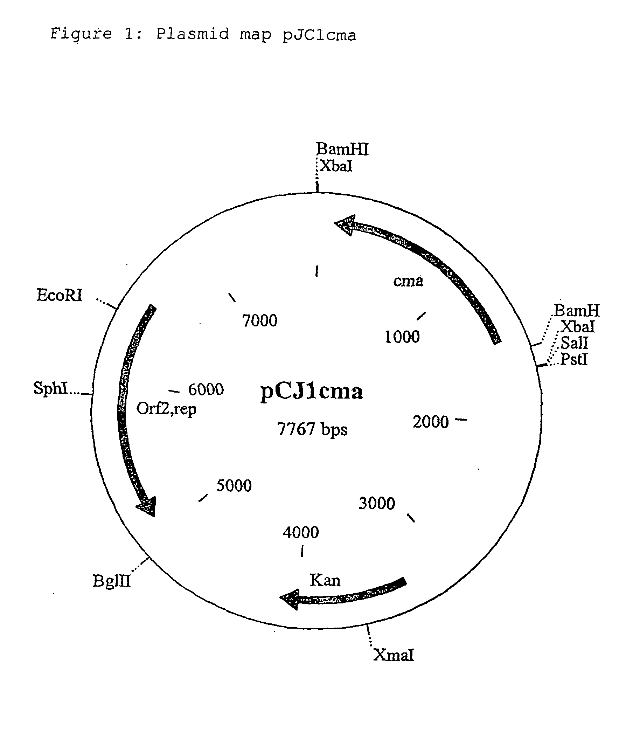

An example of a plasmid, with the aid of which the cma gene can be over-expressed is pJC1cma (figure 1), which is based on the *E. coli - C. glutamicum* shuttle vector pJC1 (Cremer et al., 1990, Molecular and General Genetics 220: 478 - 480) and contains the DNA sequence of *Corynebacterium glutamicum* which codes for the cma gene. It is contained in the strains ATCC 13032/pJC1cma and DSM5715/pJC1cma.

Plasmid vectors which are moreover suitable are those with the aid of which the process of gene amplification by integration into the chromosome can be used, as has been described, for example, by Reinscheid et al. (Applied and Environmental Microbiology 60, 126-132 (1994)) for duplication or amplification of the hom-thrB operon. In this method, the complete gene is cloned in a plasmid vector which can replicate in a host (typically *E. coli*), but not in *C. glutamicum.* Possible vectors are, for example, pSUP301 (Simon et al., Bio/Technology 1, 784-791 (1983)), pKl8mob or pKl9mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Invitrogen, Groningen, Holland; Bernard et al., Journal of Molecular Biology, 234: 534-541 (1993)) or pEMl (Schrumpf et al, 1991, Journal of Bacteriology 173:4510-4516). The plasmid vector which contains the gene to be amplified is then transferred into the desired strain of *C. glutamicum* by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross over" event, the resulting strain contains at least two copies of the gene in question.

In addition, it may be advantageous for the production of L-lysine and L-glutamate to amplify or over-express one or more enzymes of the particular biosynthesis pathway, of glycolysis, of anaplerosis, of the citric acid cycle or of amino acid export, in addition to the cma gene.

Thus, for example, for the preparation of L-lysine, one or more genes chosen from the group consisting of
- the dapA gene which codes for dihydrodipicolinate synthase (EP-B 0 197 335), or
- the dapE gene which codes for succinyl diaminopimelate desuccinylase, or
- the lysC gene which codes for a feed-back resistant aspartate kinase (Kalinowski et al. (1990), Molecular and General Genetics 224, 317-324), or
- the gap gene which codes for glyceraldehyde 3-phosphate dehydrogenase (Eikmanns (1992), Journal of Bacteriology 174:6076-6086), or
- the tpi gene which codes for triose phosphate isomerase (Eikmanns (1992), Journal of Bacteriology 174:6076-6086), or
- the pgk gene which codes for 3-phosphoglycerate kinase (Eikmanns (1992), Journal of Bacteriology 174:6076-6086), or
- the pyc gene which codes for pyruvate carboxylase (DE-A-19831609), or
- the mqo gene which codes for malate-quinone oxidoreductase (Molenaar et al., European Journal of Biochemistry 254, 395-403 (1998)), or
- the lysE gene which codes for lysine export (DE-A-195 48 222)
can be amplified, in particular over-expressed or amplified, at the same time.

Furthermore, for example, for the preparation of L-glutamate, one or more genes chosen from the group consisting of
- the gdh gene which codes for glutamate dehydrogenase (DE: 19907347.3) and/or
- the pyc gene which codes for pyruvate carboxylase (Peters-Wendisch et al.(1998), Microbiology 144: 915-927)
can be amplified, in particular over-expressed or amplified, at the same time.

In addition to amplification of the cma gene it may moreover be advantageous for the production of L-lysine to attenuate
- the pck gene which codes for phosphoenol pyruvate carboxykinase (DE 199 50 409.1, DSM 13047) and/or
- the pgi gene which codes for glucose 6-phosphate isomerase (US 09/396,478, DSM 12969)
at the same time.

In addition to amplification of the cma gene it may moreover be advantageous for the production of L-glutamate to attenuate
- the odhA gene which codes for α-ketoglutarate dehydrogenase (WO 9534672 A1 951221*), or
- the dtsR1 gene which codes for the DtsR1 protein (WO 952324 A1 950831*), or
- the dtsR2 gene which codes for the DtsR2 protein (WO 9902692A Al 990121*)
at the same time.

In addition to over-expression of the cma gene it may moreover be advantageous for the production of amino acids, in particular L-lysine and L-glutamate, to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The microorganisms prepared according to the invention can be cultured continuously or discontinuously in the batch process (batch culture) or in the fed batch (feed process) or repeated fed batch process (repetitive feed process) for the purpose of production of amino acids, in particular L-lysine. A summary of known culture methods are [sic] described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e. g. glucose, sucrose, lactose, fructose, maltose, molasses, starch and cellulose, oils and fats, such as e. g. soya oil, sunflower oil, groundnut oil and coconut fat, fatty acids, such as e. g. palmitic acid, stearic acid and linoleic acid, alcohols, such as e. g. glycerol and ethanol, and organic acids, such as e. g. acetic acid, can be used as the source of carbon. These substances can be used individually or as a mixture. Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture. Phosphoric acid, potassium dihydrogen phosphate or dipotassium hydrogen phosphate or the corresponding sodium-containing salts can be used as the source of phosphorus. The culture medium must moreover comprise salts of metals, such as e. g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e. g. fatty acid polyglycol esters, can be employed to control the development of foam. Suitable substances having a selective action, such as e. g. antibiotics, can be added to the medium to maintain the stability of plasmids. To maintain aerobic conditions, oxygen or oxygen-containing gas mixtures, such as e. g. air, are introduced into the culture. The temperature of the culture is usually 20°C to 45°C, and preferably 25°C to 40°C. Culturing is continued until a maximum of lysine has formed. This target is usually reached within 10 hours to 160 hours.

The analysis of L-lysine or L-glutamate can be carried out by anion exchange chromatography with subsequent ninhydrin derivatization, as described by Spackman et al. (Analytical Chemistry, 30, (1958), 1190).

The following microorganism has been deposited at the Deutsche Sammlung für Mikrorganismen und Zellkulturen (DSMZ = German Collection of Microorganisms and Cell Cultures, Braunschweig, Germany) in accordance with the Budapest Treaty:
- *Corynebacterium glutamicum* strain DSM5715/pJC1cma as DSM 13248

The process according to the invention is used for the fermentative preparation of amino acids, in particular L-lysine and L-glutamate.

Legend to the figure:
Figure 1: Map of the plasmid pJC1cma

The abbreviations and designations used have the following meaning:
- ORF2,rep:: Plasmid-coded replication origin *C. glutamicum* (of pHM1519)
- cma:: cma (cyclopropane-mycolic acid synthase) gene from *C. glutamicum* ATCC13032
- Kan:: Kanamycin resistance gene
- BamHI:: Cleavage site of the restriction enzyme BamHI
- XbaI:: Cleavage site of the restriction enzyme XbaI
- SalI:: Cleavage site of the restriction enzyme SalI
- PstI:: Cleavage site of the restriction enzyme PstI
- XmaI:: Cleavage site of the restriction enzyme XmaI
- BglII:: Cleavage site of the restriction enzyme BglII
- SphI:: Cleavage site of the restriction enzyme SphI
- EcoRI:: Cleavage site of the restriction enzyme EcoRI

### Examples

The present invention is explained in more detail in the following with the aid of embodiment examples.

### Example 1

### Preparation of a genomic cosmid gene library from Corynebacterium glutamicum ATCC 13032

Chromosomal DNA from *Corynebacterium glutamicum* ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vektor Kit, Code no. 251301), was cleaved with the restriction enzyme XbaI (Amersham Pharmacia, Freiburg, Germany, Product Description XbaI, Code no. 27-0948-02) and likewise dephosphorylated with shrimp alkaline phosphatase. The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC 13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extracts (Stratagene, La Jolla, USA, Product Description Gigapack II XL Packing Extract, Code no. 200217). For infection of the *E. coli* strain NM554 (Raleigh et al. 1988, Nucleic Acid Research 16:1563-1575) the cells were taken up in 10 mM MgSO₄ and mixed with an aliquot of the phage suspension. The infection and titering of the cosmid library were carried out as described-by Sambrook et al. (1989, Molecular Cloning: A laboratory Manual, Cold Spring Harbor), the cells being plated out on LB agar (Lennox, 1955, Virology, 1:190) with 100 mg/1 ampicillin. After incubation overnight at 37°C, recombinant individual clones were selected.

### Example 2

### Isolation and sequencing of the cma gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany). The DNA of the sequencing vector pZero-1, obtained from Invitrogen (Groningen, Holland, Product Description Zero Background Cloning Kit, Product No. K2500-01) was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A laboratory Manual, Cold Spring Harbor), the DNA mixture being incubated overnight with T4 ligase (Pharmacia Biotech, Freiburg, Germany). This ligation mixture was then transformed by means of electroporation (Tauch et al. 1994, FEMS Microbiol Letters, 123:343-7) into the *E. coli* strain DH5αMCR (Grant, 1990, Proceedings of the National Academy of Sciences U.S.A., 87:4645-4649) and plated out on LB agar (Lennox, 1955, Virology, 1:190) with 50 mg/1 zeocin. The plasmid preparation of the recombinant clones was carried out with Biorobot 9600 (Product No. 900200, Qiagen, Hilden, Germany). The sequencing was carried out by the dideoxy chain-stopping method of Sanger et al. (1977, Proceedings of the National Academy of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZerol derivatives were assembled to a continuous contig. The computer-assisted coding region analysis was prepared with the XNIP program (Staden, 1986, Nucleic Acids Research, 14:217-231). Further analyses were carried out with the "BLAST search program" (Altschul et al., 1997, Nucleic Acids Research, 25:3389-3402), against the non-redundant databank of the "National Center for Biotechnology Information" (NCBI, Bethesda, MD, USA).

The resulting nucleotide sequence is shown in SEQ ID No. 1. Analysis of the nucleotide sequence showed an open reading frame of 1353 base pairs, which was called the cma gene. The cma gene codes for a protein of 451 amino acids.

The following microorganism has been deposited at the Deutsche Sammlung für Mikrorganismen und Zellkulturen (DSMZ = German Collection of Microorganisms and Cell Cultures, Braunschweig, Germany) in accordance with the Budapest Treaty:
- *Corynebacterium glutamicum* strain DSM5715/pJC1cma as DSM 13248

### Example 3

### Cloning of the cma gene in the vector pJC1

Chromosomal DNA from *Corynebacterium glutamicum* ATCC 13032 was isolated as described by Tauch et al. (1995, Plasmid 33:168-179). A DNA fragment which carries the cma gene was amplified with the aid of the polymerase chain reaction. The following primers were used for this:

The two oligonucleotides carry the sequence for the cleavage site of the restriction enzyme XbaI (nucleotides underlined). The primers shown were synthesized by MWG Biotech (Ebersberg, Germany) and the PCR reaction was carried out with them by the standard PCR method of Innis et al., (PCR protocol. A guide to methods and applications, 1990, Academic Press). The primers allow amplification of a DNA fragment of 1653 bp in size, which carries the cma gene from *Corynebacterium glutamicum*.

After separation by gel electrophoresis, the PCR fragment was isolated from the agarose gel with the QiaExII Gel Extraction Kit (Product No. 20021, Qiagen, Hilden, Germany).

The PCR fragment obtained in this manner was cleaved completely with the restriction enzyme XbaI. The cma fragment approx. 1659 bp in size was isolated from the agarose gel with the QiaExII Gel- Extraction Kit (Product No. 20021, Qiagen, Hilden, Germany).

The *E. coli - C. glutamicum* shuttle vector pJC1 (Cremer et al., 1990, Molecular and General Genetics 220: 478 - 480) was used as the vector. This plasmid was also cleaved completely with the restriction enzyme XbaI and then dephosphorylated with shrimp alkaline phosphatase (Roche Diagnostics GmbH, Mannheim, Germany, Product Description SAP, Product No. 1758250).

The cma fragment obtained in this manner was mixed with the prepared vector pJC1 and the batch was treated with T4 DNA ligase (Amersham Pharmacia, Freiburg, Germany, Product Description T4-DNA-Ligase, Code no.27-0870-04). The ligation batch was transformed in the *E. coli* strain DH5α (Hanahan, In: DNA cloning. A practical approach. Vol. I. IRL-Press, Oxford, Washington DC, USA). Selection of plasmid-carrying cells was made by plating out the transformation batch on LB agar (Lennox, 1955, Virology, 1:190) with 50 mg/1 kanamycin. After incubation overnight at 37°C, recombinant individual clones were selected. Plasmid DNA was isolated from a transformant with the Qiaprep Spin Miniprep Kit (Product No. 27106, Qiagen, Hilden, Germany) in accordance with the manufacturer's instructions and cleaved with the restriction enzyme XbaI to check the plasmid by subsequent agarose gel electrophoresis. The resulting plasmid was called pJC1cma.

### Example 4:

### Transformation of the strains DSM5715 and ATCC13032 with the plasmid pJC1cma

The strains DSM5715 and ATCC13032 were transformed with the plasmid pJC1cma using the electroporation method described by Liebl et al., (FEMS Microbiology Letters, 53:299-303 (1989)). Selection of the transformants took place on LBHIS agar comprising 18.5 g/l brain-heart infusion broth, 0.5M sorbitol, 5 g/l Bacto-tryptone, 2.5 g/1 Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from in each case one transformant by conventional methods (Peters-Wendisch et al., 1998, Microbiology 144, 915 -927) and cleaved with the restriction endonuclease BamHI, to check the plasmid by subsequent agarose gel electrophoresis. The resulting strains were called DSM5715/pJC1cma and ATCC13032/pJC1cma.

The following microorganism has been deposited at the Deutsche Sammlung für Mikrorganismen und Zellkulturen (DSMZ = German Collection of Microorganisms and Cell Cultures, Braunschweig, Germany) in accordance with the Budapest Treaty:
* *Corynebacterium glutamicum* strain DSM 5715/pJClcma as DSM 13248

### Example 5:

### Preparation of L-glutamate with the strain TCC13032/pJC1cma

The *C. glutamicum* strain ATCC13032/pJC1cma obtained in example 4 was cultured in a nutrient medium suitable for the production of glutamate and the glutamate content in the culture supernatant was determined.

For this, the strain was first incubated on an agar plate with the corresponding antibiotic (brain-heart agar with kanamycin (50 mg/l)) for 24 hours at 33°C. Starting from this agar plate culture, a preculture was seeded (10 ml medium in a 100 ml conical flask). The complete medium CgIII (2.5 g/l NaCl, 10 g/l Bacto-peptone, 10 g/l Bacto-yeast extract, pH 7.4, 20 g/l Glucose (autoclaved separately) was used as the medium for the preculture. Kanamycin (25 mg/l) was added to this. The preculture was incubated for 16 hours at 33°C at 240 rpm on a shaking machine. A main culture was seeded from this preculture such that the initial OD (660nm) of the main culture was 0.1. Medium CgXII was used for the main culture.

After preculturing in medium CgIII (Keilhauer et al. 1993, Journal of Bacteriology 175:5595-5603), the strain ATCC13032/pJC1cma was cultured in the production medium CgXII (Keilhauer et al. 1993, Journal of Bacteriology 175:5595-5603). 4% glucose and 50 mg/l kanamycin sulfate were added.

For induction of the glutamate formation, 20g Tween 60 (P-1629, Sigma-Aldrich, Deisenhofen, Germany) plus 80 ml water were mixed and autoclaved. About 4 hours after the inoculation, 75 µl of this Tween solution were added to the culture and culturing was continued.

Culturing is carried out in a 10 ml volume in a 100 ml conical flask with baffles. Kanamycin (25 mg/l) was added. Culturing was carried out at 33°C and 80% atmospheric humidity.

After 48 hours, the OD was determined at a measurement wavelength of 660 nm with a Biomek 1000 (Beckmann Instruments GmbH, Munich). The amount of glutamate formed was determined with an amino acid analyzer from Eppendorf-BioTronik (Hamburg, Germany) by ion exchange chromatography and post-column derivatization with ninhydrin detection.

The result of the experiment is shown in table 1.

**Table 1**

| Strain | OD(660) | Glutamate HCl mM |
|---|---|---|
| ATCC13032/pJC1cma | 14.7 | 106 |
| ATCC13032 | 13.8 | 94 |

### Example 6:

### Preparation of lysine

The *C. glutamicum* strain DSM5715/pJC1cma obtained in example 4 was cultured in a nutrient medium suitable for the production of lysine and the lysine content in the culture supernatant was determined.

For this, the strain was first incubated on an agar plate with the corresponding antibiotic (brain-heart agar with kanamycin (50 mg/l)) for 24 hours at 33°C. Starting from this agar plate culture, a preculture was seeded (10 ml medium in a 100 ml conical flask). The complete medium CgIII (2.5 g/l NaCl, 10 g/l Bacto-peptone, 10 g/l Bacto-yeast extract, pH7.4, 20 g/l Glucose (autoclaved separately) was used as the medium for the preculture. Kanamycin (25 mg/l) was added to this. The preculture was incubated for 16 hours at 33°C at 240 rpm on a shaking machine. A main culture was seeded from this preculture such that the initial OD (660nm) of the main culture was 0.1. Medium MM was used for the main culture.

| | |
|---|---|
| Medium MM CSL (corn steep liquor) | 5 g/l |
| MOPS (morpholinopropanesulfonic acid) | 20 g/l |
| Glucose(autoclaved separately) | 50g/l |
| (NH₄)₂SO₄ | 25 g/l |
| KH₂PO₄ | 0.1 g/1 |
| MgSO₄ * 7 H₂O | 1.0 g/1 |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Kanamycin (25 µg/l) was added. Culturing was carried out at 33°C and 80% atmospheric humidity.

After 24 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in table 1.

**Table 1**

| Strain | OD(660) | Lysine HCl g/l |
|---|---|---|
| DSM5715/pJC1cma | 12.47 | 8.51 |
| DSM5715 | 11.90 | 7.80 |

## Claims

1. A genetically modified coryneform bacterium in which a polynucleotide encoding a polypeptide having an amino acid sequence which is at least 90 % identical to the amino acid sequence set out in SEQ ID NO:2 is amplified, wherein the polypeptide has the activity of cyclopropane-mycolic acid synthase, and which produce L-lysin or L-glutamate in an improved manner in relation to the corresponding non-genetically modified coryneform bacterium.

2. The bacterium of claim 1, wherein said polynucleotide is overexpressed.

3. The bacterium of claim 1, wherein said polypeptide has the amino acid sequence set out in SEQ ID NO:2.

4. Coryneform bacterium according to claim 1 or 2, especially of genus Corynebacterium glutamicum, wherein the polynucleotide is chosen from the group:
a) polynucleotide having the nucleotide sequence as set out in SEQ ID NO:1;
b) polynucleotides having nucleotide sequences corresponding to sequence SEQ ID NO:1 within the degeneracy of the genetic code.

5. Corynebacterium glutamicum, deposited under number DSM 13248.

6. Coryneform bacterium according to claim 2, wherein the amplification of the cma gene is carried out by over-expression of the gene, in particular by increasing the number of copies of the gene, by choosing a potent promoter or a regulation region upstream the reading frame, by mutation of the promoter, the regulation region or the ribosome binding site, by incorporation of a suitable expression cassette upstream the structural gene or by incorporation of inducible promoters, by prolonging the life of the corresponding mRNA, by a reduced degradation of the proteins expressed, or by combination of several of these possibilities.

7. A process for the preparation of L-lysine or L-glutamate comprising:
fermentation of said amino acid producing bacterium according to any of the claims 1 to 6.

8. A process according to claim 7, comprising:
a) fermentation of said coryneform bacteria which produce the desired L-amino acid,
b) concentration of said amino acid in the medium or in the cells of the bacteria and
c) isolation of the L-amino acid.

9. The process according to claims 7 to 8, wherein bacteria are employed in which further genes of the biosynthesis pathway of L-lysine or L-glutamate are additionally amplified.

10. The process according to claims 7 to 9, wherein bacteria are employed in which the metabolic pathways which reduce the formation of L-lysine or L-glutamate are eliminated.

11. A process according to claim 9,
**wherein**
for the preparation of L-lysine or L-glutamate, bacteria are fermented in which at the same time one or more genes chosen from the group consisting of
a) the dapA gene which codes for dihydrodipicolinate synthase,
b) the dapE gene which codes for succinyl diaminopimelate desuccinylase,
c) the lysC gene which codes for a feed-back resistant aspartate kinase,
d) the tpi gene which codes for triose phosphate isomerase,
e) the gap gene which codes for glyceraldehyde 3-phosphate dehydrogenase,
f) the pgk gene which codes for 3-phosphoglycerate kinase,
g) the pyc gene which codes for pyruvate carboxylase,
h) the mqo gene which codes for malate:quinone oxidoreductase,
i) the lysE gene which codes for a protein for lysine export,
is or are amplified, in particular over-expressed, at the same time.

12. A process as claimed in one of claims 9 to 11,
**wherein**
for the preparation of L-lysine, bacteria are fermented in which one or more genes chosen from the group consisting of
a) the pck gene which codes for phosphoenol pyruvate carboxykinase
b) the pgi gene which codes for glucose 6-phosphate isomerase
is attenuated at the same time.

13. A process according to claim 9, wherein for the preparation of L-glutamate bacteria are fermented in which one or more of the genes chosen from the group consisting of:
a) the gdh gene which codes for glutamate dehydrogenase or
b) the pyc gene which codes for pyruvate carboxylase are amplified, in particular over-expressed at the same time.

14. A process according to claim 10 wherein for the preparation of L-glutamate, bacteria are fermented in which one or more genes chosen from the group consisting of
a) the odhA gene which codes for α-ketoglutarate dehydrogenase
b) the dtsR1 gene which codes for the DtsR1 protein,
c) the dtsR2 gene which codes for the DtsR2 protein are attenuated at the same time.

## Patentansprüche

1. Gentechnisch verändertes coryneformes Bakterium, in dem ein für ein Polypeptid mit einer Aminosäuresequenz, die mindestens zu 90% mit der in SEQ ID NO:2 angegebenen Aminosäuresequenz identisch ist, codierendes Polynukleotid verstärkt ist, wobei das Polypeptid die Aktivität einer Cyclopropan-Mycolsäure-Synthase aufweist, wobei das Bakterium L-Lysin oder L-Glutamin auf verbesserte Weise im Verhältnis zu dem entsprechenden nicht gentechnisch veränderten coryneformen Bakterium produziert.

2. Bakterium nach Anspruch 1, wobei das Polynukleotid überexprimiert ist.

3. Bakterium nach Anspruch 1, wobei das Polypeptid die in SEQ ID NO:2 angegebene Aminosäuresequenz aufweist.

4. Coryneformes Bakterium gemäß Anspruch 1 oder 2, insbesondere der Gattung Corynebacterium glutamicum, wobei das Polynukleotid ausgewählt ist aus der Gruppe:
a) Polynukleotid mit der wie in SEQ ID N0:1 angegebenen Nukleotidsequenz;
b) Polynukleotide mit Nukleotidsequenzen, die der Sequenz SEQ ID NO:1 im Rahmen der Degeneration des genetischen Codes entsprechen.

5. Corynebacterium glutamicum, hinterlegt unter der Nummer DSM 13248.

6. Coryneformes Bakterium gemäß Anspruch 2, wobei die Verstärkung des cma-Gens durch Überexpression des Gens, insbesondere durch Erhöhung der Kopienzahl des Gens, durch die Wahl eines starken Promotors oder einer Regulationsregion oberhalb des Leserasters, durch Mutation des Promotors, der Regulationsregion oder der Ribosomenbindungsstelle, durch Einbau einer geeigneten Expressionskassette oberhalb des Strukturgens oder durch Einbau von induzierbaren Promotoren, durch die Verlängerung der Lebensdauer der entsprechenden mRNA, durch einen verminderten Abbau der exprimierten Proteine oder durch die Kombination mehrerer dieser Möglichkeiten erfolgt.

7. Verfahren zur Herstellung von L-Lysin oder L-Glutamat, bei dem man:
eine Fermentation des aminosäureproduzierenden Bakteriums gemäß einem der Ansprüche 1 bis 6 durchführt.

8. Verfahren gemäß Anspruch 7, bei dem man:
a) eine Fermentation der die gewünschte L-Aminosäure produzierenden coryneformen Bakterien,
b) eine Anreicherung der Aminosäure im Medium oder in den Zellen der Bakterien und
c) eine Isolierung der L-Aminosäure durchführt.

9. Verfahren gemäß Anspruch 7 und 8, wobei man Bakterien einsetzt, in denen zusätzlich weitere Gene des Biosyntheseweges von L-Lysin oder L-Glutamat verstärkt sind.

10. Verfahren gemäß Anspruch 7 bis 9, wobei man Bakterien einsetzt, in denen die Stoffwechselwege, die die Bildung von L-Lysin oder L-Glutamat verringern, ausgeschaltet sind.

11. Verfahren gemäß Anspruch 9, wobei man für die Herstellung von L-Lysin oder L-Glutamat Bakterien fermentiert, in denen gleichzeitig eines oder mehrere der Gene, ausgewählt aus der Gruppe:
a) das für die Dihydrodipicolinat-Synthase codierende dapA-Gen,
b) das für die Succinyldiaminopimelat-Desuccinylase codierende dapE-Gen,
c) das für eine feedback-resistente Aspartatkinase codierende lysC-Gen,
d) das für die Triosephosphat-Isomerase codierende tpi-Gen,
e) das für die Glyceraldehyd-3-phosphat-Dehydrogenase codierende gap-Gen,
f) das für die 3-Phosphoglycerat-Kinase codierende pgk-Gen,
g) das für die Pyruvat-Carboxylase codierende pyc-Gen,
h) das für die Malat:Chinon-Oxidoreduktase codierende mqo-Gen,
i) das für ein Protein für den Lysin-Export codierende lysE-Gen,
gleichzeitig verstärkt, insbesondere überexprimiert, ist bzw. sind.

12. Verfahren nach einem der Ansprüche 9 bis 11, wobei man für die Herstellung von L-Lysin Bakterien fermentiert, in denen gleichzeitig ein Gen oder mehrere Gene, ausgewählt aus der Gruppe:
a) das für die Phosphoenolpyruvat-Carboxykinase codierende pck-Gen,
b) das für die Glucose-6-phosphat-Isomerase codierende pgi-Gen,
abgeschwächt sind.

13. Verfahren gemäß Anspruch 9, wobei man für die Herstellung von L-Glutamat Bakterien fermentiert, in denen gleichzeitig eines oder mehrere der Gene, ausgewählt aus der Gruppe:
a) das für die Glutamat-Dehydrogenase codierende gdh-Gen,
b) das für die Pyruvat-Carboxylase codierende pyc-Gen, verstärkt, insbesondere überexprimiert, sind.

14. Verfahren gemäß Anspruch 10, wobei man für die Herstellung von L-Glutamat Bakterien fermentiert, in denen gleichzeitig ein Gen oder mehrere Gene, ausgewählt aus der Gruppe:
a) das für die α-Ketoglutarat-Dehydrogenase codierende odhA-Gen,
b) das für das DtsR1-Protein codierende dtsR1-Gen,
c) das für das DtsR2-Protein codierende dtsR2-Gen,
abgeschwächt sind.

## Revendications

1. Bactérie corynéforme génétiquement modifiée dans laquelle un polynucléotide codant pour un polypeptide présentant une séquence d'acides aminés qui est identique au moins à 90% à la séquence d'acides aminés décrite à la SEQ ID NO:2 est amplifié, dans laquelle le polypeptide présente l'activité d'une acide cyclopropane-mycolique synthase, et qui produit de la L-lysine ou du L-glutamate d'une manière améliorée par rapport à la bactérie du genre Corynebacterium correspondante non génétiquement modifiée.

2. Bactérie suivant la revendication 1, dans laquelle ledit polynucléotide est surexprimé.

3. Bactérie suivant la revendication 1, dans laquelle ledit polypeptide présente la séquence d'acides aminés décrite à la SEQ ID NO:2.

4. Bactérie corynéforme suivant la revendication 1 ou 2, spécialement du genre Corynebacterium glutamicum dans laquelle le polynucléotide est choisi parmi le groupe comprenant :
a) un polynucléotide présentant la séquence nucléotidique telle que décrite à la SEQ ID NO:1;
b) des polynucléotides présentant des séquences nucléotidiques correspondant à la SEQ ID NO:1 dans le cadre de la dégénérescence du code génétique.

5. Corynebacterium glutamicum déposée sous le numéro DSM 13 248.

6. Bactérie corynéforme suivant la revendication 2, dans laquelle l'amplification du gène cma est réalisée par une surexpression du gène, en particulier par une augmentation du nombre de copies du gène, par le choix d'un promoteur puissant ou d'une région de régulation en amont du cadre de lecture, par une mutation du promoteur, de la région de régulation ou du site de liaison au ribosome, par une incorporation d'une cassette appropriée d'expression en amont du gène structurel ou par une incorporation de promoteurs inductibles, par une prolongation de la vie des ARNm correspondants, par une dégradation réduite des protéines exprimées ou par une combinaison de plusieurs de ces possibilités.

7. Procédé de préparation de L-lysine ou de L-glutamate comprenant :
la fermentation de ladite bactérie produisant les acides aminés suivant l'une quelconque des revendications 1 à 6.

8. Procédé suivant la revendication 7, comprenant :
a) la fermentation desdites bactéries corynéformes qui produisent le L-acide aminé souhaité,
b) la concentration dudit acide aminé dans le milieu ou dans les cellules des bactéries et
c) l'isolement de l'acide aminé L.

9. Procédé suivant les revendications 7 à 8, dans lequel on utilise des bactéries dans lesquelles d'autres gènes de la voie de biosynthèse de la L-lysine ou du L-glutamate sont en outre amplifiés.

10. Procédé suivant les revendications 7 à 9, dans lequel on utilise des bactéries dans lesquelles les voies métaboliques qui réduisent la formation de la L-lysine ou du L-glutamate sont éliminées.

11. Procédé suivant la revendication 9, dans lequel pour la préparation de la L-lysine ou du L-glutamate, on met en fermentation des bactéries dans lesquelles au même moment un ou plusieurs gènes choisis parmi le groupe comprenant :
a) le gène dapA qui code pour la dihydrodipicolinate synthase,
b) le gène dapE qui code pour la succinyldiaminopimélate désuccinylase,
c) le gène lysC qui code pour une aspartate kinase résistante à une rétroaction,
d) le gène tpi qui code pour la triose-phosphate isomérase,
e) le gène gap qui code pour la glycéraldéhyde-3-phosphate déshydrogénase,
f) le gène pgk qui code pour la 3-phosphoglycérate kinase,
g) le gène pyc qui code pour la pyruvate carboxylase,
h) le gène mqo qui code pour la malate:quinone oxydoréductase,
i) le gène lysE qui code pour une protéine destinée à l'export de la lysine,
est ou sont amplifiés, en particulier surexprimés, au même moment.

12. Procédé suivant l'une des revendications 9 à 11, dans lequel pour la préparation de la L-lysine, des bactéries sont mises en fermentation dans lesquelles un ou plusieurs gènes choisis parmi le groupe comprenant :
a) le gène pck qui code pour la phosphoénolpyruvate carboxykinase,
b) le gène pgi qui code pour la glucose-6-phosphate isomérase,
est ou sont atténués au même moment.

13. Procédé suivant la revendication 9, dans lequel, pour la préparation du L-glutamate, des bactéries sont mises en fermentation dans lesquelles un ou plusieurs des gènes choisis parmi le groupe comprenant :
a) le gène gdh qui code pour la glutamate déshydrogénase ou
b) le gène pyc qui code pour la pyruvate carboxylase
est ou sont amplifiés, en particulier surexprimés au même moment.

14. Procédé suivant la revendication 10, dans lequel pour la préparation du L-glutamate, on met en fermentation des bactéries dans lesquelles un ou plusieurs gènes choisis parmi le groupe comprenant :
a) le gène odhA qui code pour l'α-cétoglutarate déshydrogénase,
b) le gène dtsR1 qui code pour la protéine DtsR1,
c) le gène dtsR2 qui code pour la protéine DtsR2
est ou sont atténués au même moment.


### Sequence protocol

<110> Degussa-Hüls AG, Forschungszentrum Jülich GmbH
<120> New nucleotide sequences which code for the cma gene
<130> 000007 BT
<140>
   <141>
<160> 2
<170> PatentIn Ver. 2.1
<210> 1
   <211> 1801
   <212> DNA
   <213> Corynebacterium glutamicum
<220>
   <221> CDS
   <222> (248)..(1600)
   <223> cma-Gen
<400> 1
   ctccgatgca ccagagggag catttaacag agaaatcgaa cgcgtcatgg cagagctagg 60
   cggacataaa tcgctgtact ccgaagcgtt ctacaccagg gaagactttg aaaaacttta 120
   tggcggaacc atcccggcgc tgctaaaaaa gcagtgggat ccccacagcc gattccccgg 180
   tttgtatgaa aagacagtaa aaggcgccta ggatcgctca ctgtaggtag aggcttgtgg 240
   tcactac ttg tgg cca cat ttt aaa aaa atg cac aag aag aga aag caa    289
           Leu Trp Pro His Phe Lys Lys Met His Lys Lys Arg Lys Gln
             1               5                  10
   agc att atg agt aac gcc gta gcg cag gac ctc atg acc atc gcc gac    337
   Ser Ile Met Ser Asn Ala Val Ala Gln Asp Leu Met Thr Ile Ala Asp
    15                  20                  25                  30
   atc gtc gag gcc acg acc act gca ccc atc cca ttc cac atc act gcc    385
   Ile Val Glu Ala Thr Thr Thr Ala Pro Ile Pro Phe His Ile Thr Ala
                    35                  40                  45
   ttc gat gga agc ttc act ggc cct gaa gat gct ccc tac cag ctg ttt    433
   Phe Asp Gly Ser Phe Thr Gly Pro Glu Asp Ala Pro Tyr Gln Leu Phe
                50                  55                  60
   gtt gcc aac acg gat gca gta tcc tac atc gca aca gcg cca gga gat    481
   Val Ala Asn Thr Asp Ala Val Ser Tyr Ile Ala Thr Ala Pro Gly Asp
            65                  70                  75
   ttg ggt ttg gca cgt gcc tac ctc atg gga gac ctc atc gtg gaa ggt    529
   Leu Gly Leu Ala Arg Ala Tyr Leu Met Gly Asp Leu Ile Val Glu Gly
        80                  85                  90
   gag cat ccc ggc cat cct tat ggg atc ttt gat gcg ttg aag gag ttc    577
   Glu His Pro Gly His Pro Tyr Gly Ile Phe Asp Ala Leu Lys Glu Phe
    95                 100                 105                 110
   tac cgc tgc ttc aaa cgc cca gat gca tcc acc acc ttg cag atc atg     625
   Tyr Arg Cys Phe Lys Arg Pro Asp Ala Ser Thr Thr Leu Gln Ile Met
                   115                 120                 125
   tgg act ctg cgg aaa atg aat gcc tta aaa ttc cag gaa att cca cca     673
   Trp Thr Leu Arg Lys Met Asn Ala Leu Lys Phe Gln Glu Ile Pro Pro
               130                 135                 140
   atg gaa caa gcc cct gca tgg cgt aaa gca ctg atc aac ggg cta gca     721
   Met Glu Gln Ala Pro Ala Trp Arg Lys Ala Leu Ile Asn Gly Leu Ala
           145                 150                 155
   tcc agg cac tcg aaa tcc cgc gac aag aaa gcc att agc tac cac tac     769
   Ser Arg His Ser Lys Ser Arg Asp Lys Lys Ala Ile Ser Tyr His Tyr
       160                 165                 170
   gac gtg ggc aat gag ttc tac tcc ctg ttt tta gat gat tcc atg acc     817
   Asp Val Gly Asn Glu Phe Tyr Ser Leu Phe Leu Asp Asp Ser Met Thr
   175                 180                 185                 190
   tat acc tgc gcg tat tat cca acg cca gaa tca agt ttg gaa gaa gcc     865
   Tyr Thr Cys Ala Tyr Tyr Pro Thr Pro Glu Ser Ser Leu Glu Glu Ala
                   195                 200                 205
   caa gaa aac aaa tac cgc ctc atc ttt gaa aaa ctg cgt ctg aaa gaa     913
   Gln Glu Asn Lys Tyr Arg Leu Ile Phe Glu Lys Leu Arg Leu Lys Glu
               210                 215                 220
   ggc gat cgc ctc cta gac gtg gga tgc ggt tgg gga ggc atg gtc cgc     961
   Gly Asp Arg Leu Leu Asp Val Gly Cys Gly Trp Gly Gly Met Val Arg
           225                 230                 235
   tac gcc gcc aaa cac ggt gtg aaa gcc atc gga gtt acg ctg tct gaa    1009
   Tyr Ala Ala Lys His Gly Val Lys Ala Ile Gly Val Thr Leu Ser Glu
       240                 245                 250
   cag caa tat gag tgg ggt caa gca gag atc aaa cgc caa ggt ttg gaa    1057
   Gln Gln Tyr Glu Trp Gly Gln Ala Glu Ile Lys Arg Gln Gly Leu Glu
   255                 260                 265                 270
   gac ctc gcg gaa att cgc ttc atg gat tac cgc gat gtt cca gaa act    1105
   Asp Leu Ala Glu Ile Arg Phe Met Asp Tyr Arg Asp Val Pro Glu Thr
                   275                 280                 285
   gga ttc gat gcg atc tca gca atc ggc atc att gaa cac atc ggt gtg    1153
   Gly Phe Asp Ala Ile Ser Ala Ile Gly Ile Ile Glu His Ile Gly Val
               290                 295                 300
   aac aac tat ccc gac tac ttt gaa ttg ctc agc agc aaa ctc aaa aca    1201
   Asn Asn Tyr Pro Asp Tyr Phe Glu Leu Leu Ser Ser Lys Leu Lys Thr
           305                 310                 315
   ggc gga ctg atg ctc aac cac agc atc acc tac cca gac aac cgc ccc    1249
   Gly Gly Leu Met Leu Asn His Ser Ile Thr Tyr Pro Asp Asn Arg Pro
       320                 325                 330
   cgc cac gca ggt gca ttt att gat cgc tac att ttc ccc gac ggt gaa    1297
   Arg His Ala Gly Ala Phe Ile Asp Arg Tyr Ile Phe Pro Asp Gly Glu
   335                 340                 345                 350
   ctc act ggc tct ggc acc ctg atc aag cac atg cag gac aac ggt ttc    1345
   Leu Thr Gly Ser Gly Thr Leu Ile Lys His Met Gln Asp Asn Gly Phe
                   355                 360                 365
   gaa gtg ctg cac gaa gaa aac ctc cgc ttt gat tac caa cgc acc ctg    1393
   Glu Val Leu His Glu Glu Asn Leu Arg Phe Asp Tyr Gln Arg Thr Leu
               370                 375                 380
   cac gcg tgg tgc gaa aac ctc aaa gaa aat tgg gag gaa gca gtt gaa    1441
   His Ala Trp Cys Glu Asn Leu Lys Glu Asn Trp Glu Glu Ala Val Glu
           385                 390                 395
   ctc gcc ggt gaa ccc act gca cga ctc ttt ggc ctg tac atg gca ggt    1489
   Leu Ala Gly Glu Pro Thr Ala Arg Leu Phe Gly Leu Tyr Met Ala Gly
       400                 405                 410
   tcg gaa tgg gga ttt gcc cac aac atc gtc cag ctg cac caa gta ctg    1537
   Ser Glu Trp Gly Phe Ala His Asn Ile Val Gln Leu His Gln Val Leu
   415                 420                 425                 430
   ggt gtg aaa ctc gat gag cag gga agt cgc gga gaa gtt cct gaa aga    1585
   Gly Val Lys Leu Asp Glu Gln Gly Ser Arg Gly Glu Val Pro Glu Arg
                   435                 440                 445
   atg tgg tgg act atc taaagaaaca atgtttcttt ttaaggttcc ctaggggcag    1640
   Met Trp Trp Thr Ile
               450
   gagttaattg taggtaaact cgactagcaa ttagcgactg tcccatttcc cacctgcttt 1700
   tgcggatttc aacactttca ggatgcatct gtaactatgc ccatgccaca ccccatgttt 1760
   atcgatgtct ccttcccacc cgcggagacc tgtagttaac c                     1801
<210> 2
   <211> 451
   <212> PRT
   <213> Corynebacterium glutamicum
<400> 2
   Leu Trp Pro His Phe Lys Lys Met His Lys Lys Arg Lys Gln Ser Ile
    1               5                  10                  15
   Met Ser Asn Ala Val Ala Gln Asp Leu Met Thr Ile Ala Asp Ile Val
               20                  25                  30
   Glu Ala Thr Thr Thr Ala Pro Ile Pro Phe His Ile Thr Ala Phe Asp
           35                  40                  45
   Gly Ser Phe Thr Gly Pro Glu Asp Ala Pro Tyr Gln Leu Phe Val Ala
       50                  55                  60
   Asn Thr Asp Ala Val Ser Tyr Ile Ala Thr Ala Pro Gly Asp Leu Gly
   65                  70                  75                  80
   Leu Ala Arg Ala Tyr Leu Met Gly Asp Leu Ile Val Glu Gly Glu His
                   85                  90                  95
   Pro Gly His Pro Tyr Gly Ile Phe Asp Ala Leu Lys Glu Phe Tyr Arg
               100                 105                 110
   Cys Phe Lys Arg Pro Asp Ala Ser Thr Thr Leu Gln Ile Met Trp Thr
   115 120 125
   Leu Arg Lys Met Asn Ala Leu Lys Phe Gln Glu Ile Pro Pro Met Glu
   130 135 140
   Gln Ala Pro Ala Trp Arg Lys Ala Leu Ile Asn Gly Leu Ala Ser Arg
   145 150 155 160
   His Ser Lys Ser Arg Asp Lys Lys Ala Ile Ser Tyr His Tyr Asp Val
   165 170 175
   Gly Asn Glu Phe Tyr Ser Leu Phe Leu Asp Asp Ser Met Thr Tyr Thr
   180 185 190
   Cys Ala Tyr Tyr Pro Thr Pro Glu Ser Ser Leu Glu Glu Ala Gln Glu
   195 200 205
   Asn Lys Tyr Arg Leu Ile Phe Glu Lys Leu Arg Leu Lys Glu Gly Asp
   210 215 220
   Arg Leu Leu Asp Val Gly Cys Gly Trp Gly Gly Met Val Arg Tyr Ala
   225 230 235 240
   Ala Lys His Gly Val Lys Ala Ile Gly Val Thr Leu Ser Glu Gln Gln
   245 250 255
   Tyr Glu Trp Gly Gln Ala Glu Ile Lys Arg Gln Gly Leu Glu Asp Leu
   260 265 270
   Ala Glu Ile Arg Phe Met Asp Tyr Arg Asp Val Pro Glu Thr Gly Phe
   275 280 285
   Asp Ala Ile Ser Ala Ile Gly Ile Ile Glu His Ile Gly Val Asn Asn
   290 295 300
   Tyr Pro Asp Tyr Phe Glu Leu Leu Ser Ser Lys Leu Lys Thr Gly Gly
   305 310 315 320
   Leu Met Leu Asn His Ser Ile Thr Tyr Pro Asp Asn Arg Pro Arg His
   325 330 335
   Ala Gly Ala Phe Ile Asp Arg Tyr Ile Phe Pro Asp Gly Glu Leu Thr
   340 345 350
   Gly Ser Gly Thr Leu Ile Lys His Met Gln Asp Asn Gly Phe Glu Val
   355 360 365
   Leu His Glu Glu Asn Leu Arg Phe Asp Tyr Gln Arg Thr Leu His Ala
       370                 375                 380
   Trp Cys Glu Asn Leu Lys Glu Asn Trp Glu Glu Ala Val Glu Leu Ala
   385                 390                 395                 400
   Gly Glu Pro Thr Ala Arg Leu Phe Gly Leu Tyr Met Ala Gly Ser Glu
                   405                 410                 415
   Trp Gly Phe Ala His Asn Ile Val Gln Leu His Gln Val Leu Gly Val
               420                 425                 430
   Lys Leu Asp Glu Gln Gly Ser Arg Gly Glu Val Pro Glu Arg Met Trp
           435                 440                 445
   Trp Thr Ile
       450